# EUROPEAN PATENT SPECIFICATION

(11) **EP 1 959 864 B1**
(45) Date of publication and mention of the grant of the patent: **07.03.2018**
(21) Application number: 06840136.3
(22) Date of filing: 06.12.2006
(51) Int. Cl.: A61F 2/24

(54) **CONNECTION SYSTEMS FOR TWO PIECE PROSTHETIC HEART VALVE ASSEMBLIES**
VERBINDUNGSSYSTEME FÜR ZWEISTÜCKIGE PROTHETISCHE HERZKLAPPENANORDNUNGEN
SYSTÈMES DE CONNEXION POUR ENSEMBLES VALVE CARDIAQUE PROSTHÉTIQUE EN DEUX PARTIES

(30) Priority: 07.12.2005 US 748639 P
(43) Date of publication of application: 27.08.2008
(73) Proprietor: Medtronic, Inc., Fridley, MN 55432 (US)
(72) Inventor: GURSKIS, Donnell, W., Belmont, CA 94002 (US); LANE, Ernest, Huntington Beach, CA 92646 (US); INO, Takashi, Harry, San Jose, CA 95123 (US)
(74) Representative: Zimmermann & Partner Patentanwälte mbB
(86) International application number: PCT/US2006/061715
(87) International publication number: WO 2007/067942

(56) References cited:
- WO-A-01/82840
- WO-A2-00/44311
- US-A1- 2004 044 406
- US-A1- 2004 122 514

## Description

### FIELD OF THE INVENTION

The present invention relates to a prosthesis for receiving a prosthetic valve.

### BACKGROUND

Prosthetic heart valves can replace defective human valves in patients. For example, one piece valves have been suggested that include sewing rings or suture cuffs that are attached to and extend around the outer circumference of a prosthetic valve. In addition, multiple component valves have also been suggested that include a sewing ring that is separate from a valve component. The sewing rings of either type of prosthetic valve can be tedious and time consuming to secure within a target site, i.e., within an annulus of a heart where a natural heart valve has been removed.

For example, to implant a sewing ring within an annulus of a heart, between twelve and twenty sutures may be secured initially to tissue surrounding the annulus. The sewing ring and/or the entire prosthetic valve may then be advanced or "parachuted" down the sutures into the annulus. Knots may then be tied with the sutures to secure the sewing ring within the annulus, whereupon the sutures may be cut. Consequently, this procedure can be very complicated, requiring management and manipulation of many sutures. The complexity of the procedure also provides a greater opportunity for mistakes and requires a patient to be on cardiopulmonary bypass for a lengthy period of time.

Because the annulus of the heart may not match the circular cross-section of the sewing ring and/or prosthetic valve, the prosthetic valve may not fit optimally within the annulus. As a result, natural blood hemodynamics through and around the valve may be impaired, resulting in clotting, possible emboli production, and eventual calcification of the valve structure.

To address this concern, flexible sewing rings have been suggested for use with multiple component valves. The sewing ring may be implanted within the annulus, e.g., using the procedure described above, i.e., parachuted down an arrangement of sutures.
The sewing ring may conform at least partially to the anatomy of the annulus. Alternatively, instead of using sutures, it has also been suggested to drive staples through the sewing ring into the surrounding tissue to secure the sewing ring.

When a mechanical or prosthetic valve is then attached to the sewing ring, however, the valve and sewing ring may not mate together effectively, e.g., if the shape of the sewing ring has been distorted to conform to the annulus, which may also impair natural blood hemodynamics, create leaks, and/or otherwise impair performance of the prosthetic valve.

US 2004/0122514 A1 describes a biologically implantable prosthesis according to the preamble of claim 1 and methods of using the same.

### SUMMARY OF THE INVENTION

The present invention is directed to heart valves that may be implanted within a patient, and, more particularly, to multiple component heart valve assemblies that may be assembled together. In accordance with one embodiment, a prosthesis is provided for receiving a prosthetic valve to replace a preexisting natural or prosthetic heart valve within a biological annulus adjacent a sinus cavity. The prosthesis includes an annular member implantable within the biological annulus, a sewing cuff extending radially outwardly from the annular member and a plurality of guide rails including a first end attached to the annular member or sewing cuff and a second free end. The guide rails include one or more locking tabs. Optionally, the guide rails may include weakened regions, e.g., above the one or more connectors, to facilitate severing the guide rails after securing a prosthetic valve to the prosthesis.

In accordance with still another embodiment, a heart valve assembly is provided that includes a first prosthesis and a second valve prosthesis. The first prosthesis includes an annular member implantable within a biological annulus, a sewing cuff extending from the annular member, and a plurality of guide rails attached to one of the annular member or sewing cuff. The second prosthesis may include an annular frame, at least one valve element and receptacles for receiving respective guide rails such that the second prosthesis may be directed along the guide rails towards the first prosthesis. One or more locking tabs are provided on the guide rails for engaging respective windows in the receptacles for securing the second prosthesis relative to the first prosthesis.

In accordance with still another example for understanding the invention, a heart valve assembly is provided that includes a first annular prosthesis including an annular member implantable within a biological annulus, a sewing cuff extending outwardly from the annular member, and a plurality of connectors extending inwardly from the sewing cuff; and a second valve prosthesis including an annular frame including a plurality of receptacles, e.g., windows, for receiving respective connectors therein to secure the second valve prosthesis relative to the first prosthesis,

In accordance with yet another example for understanding the invention, a method is provided for implanting a prosthetic heart valve assembly within a biological annulus. An annular prosthesis is provided that includes an annular member and a plurality of guide rails extending from the annular member. The annular prosthesis may be directed towards the biological annulus, e.g., until the annular member is introduced into the biological annulus. One or more connectors, e.g., sutures, clips, and the like, may be directed through a portion of the annulus prosthesis, e.g., through a sewing cuff or skirt extend radially from the annular member, to secure the annular member within the biological annulus.

A valve prosthesis, e.g., a mechanical or bioprosthetic valve, may be advanced over the guide rails, and secured relative to the annular member. For example, the valve prosthesis may include a plurality receptacles for receiving respective guide rails therethrough, such that the valve prosthesis is parachuted down the guide rails towards the annular prosthesis. The guide rails may include one or more connectors, e.g., buttons, detents, beveled surfaces, and the like, that may be received in the receptacles, e.g., in pockets in the receptacles, for securing the valve prosthesis to or adjacent the annular prosthesis. The guide rails may then be removed, e.g., by severing the guide rails above the receptacles and connectors, leaving the valve prosthesis secured to annular prosthesis adjacent the biological annulus,

In accordance with still another example for understanding the invention, a method is provided for implanting a heart valve assembly in a biological annulus that includes inserting a first annular prosthesis into the biological annulus, the first prosthesis including a plurality of guide rails extending therefrom; securing the first prosthesis to tissue surrounding the biological annulus; directing free ends of the guide rails through respective receptacles on a second valve prosthesis; and advancing the second valve prosthesis along the guide rails until connectors on the guide rails are engaged with the receptacles.

In accordance with yet another example, a method is provided for implanting a heart valve assembly in a biological annulus that includes inserting a first prosthesis into the biological annulus, the first prosthesis including a plurality of connectors extending an inner surface of a sewing cuff of the first prosthesis; securing the first prosthesis to tissue surrounding the biological annulus; and advancing a second valve prosthesis towards the implanted prosthesis until a portion of the second valve prosthesis is captured by the connectors.

Other aspects and features of the present invention will become apparent from consideration of the following description taken in conjunction with the accompanying drawings.

### BRIEF DESCRIPTION OF THE DRAWINGS

The drawings illustrate exemplary embodiments of the invention and examples for understanding the invention, in which:
FIG. 1A is a perspective view of an exemplary embodiment of a gasket member for a two piece heart valve assembly having guide rails extending therefrom.
FIG. 1B is a side view of an exemplary embodiment of a guide rail that may be provided with the gasket member of FIG. 1A.
FIG. 1C is a side view showing an alternative embodiment of a guide rail.
FIG. 2A is a perspective view of an exemplary embodiment of a valve member for a two piece heart valve assembly including receptacles for receiving guide rails.
FIG. 2B is a perspective detail showing an exemplary embodiment of a receptacle for the valve member in FIG. 2A.
FIG. 3A is a perspective view of a valve member being secured to a gasket member by a connector on a guide rail being locked into a receptacle of FIG. 2A and 2B.
FIG. 3B is a perspective view of a frame for a valve prosthesis, such as that shown in FIGS. 2A and 2B, showing a back portion of a receptacle receiving the guide rail of FIG. 1B.
FIGS. 4A and 4B are perspective views showing the operation of the guide rails and receptacles of FIGS. 1B, 2B, and 3B to provide a guiding and locking system.
FIGS. 5A and 5B are side and perspective views, respectively, showing additional features of the guiding and locking system of FIGS. 4A and 4B.
FIGS. 6A and 6B are a perspective views of a biological annulus, showing a method for implanting the gasket member of FIG. 1A and the valve member of FIG. 2A within the biological annulus.
FIGS. 7 and 8 are perspective and side views, respectively, of another embodiment of a frame for a valve member including a plurality of receptacles extending from the frame.
FIG. 9 is a perspective view of a sewing cuff core for a gasket member.
FIGS. 10A and 10B arc perspective views of components of a connector that may be attached to the sewing cuff core of FIG. 9.
FIGS. 11 and 12 are perspective and top views, respectively, of the valve frame of FIGS. 7 and 8 being secured to the sewing cuff core of FIG. 9 when connectors on the sewing cuff core are received in corresponding receptacles in the valve frame.
FIG. 13 is a perspective view of another example for understanding the invention of a valve frame and sewing cuff core of a heart valve assembly, the sewing cuff core including connectors having an eagle's beak shape secured within receptacles of the valve frame.
FIGS. 14A-14D are bottom, rear, side, and perspective views, respectively of one of the connectors of the heart valve assembly of FIG. 13.
FIG. 15A is a cross-sectional view of yet another example for understanding the invention of a heart valve assembly including a connector on a gasket member for reception in a receptacle on a valve member.
FIG. 15B is a detail of the valve member of FIG. 15A, showing a ring defining a receptacle for engaging a connector on a gasket member.
FIGS. 16A-16C, are front, side, and perspective views, respectively, of the connector of FIG. 15A.

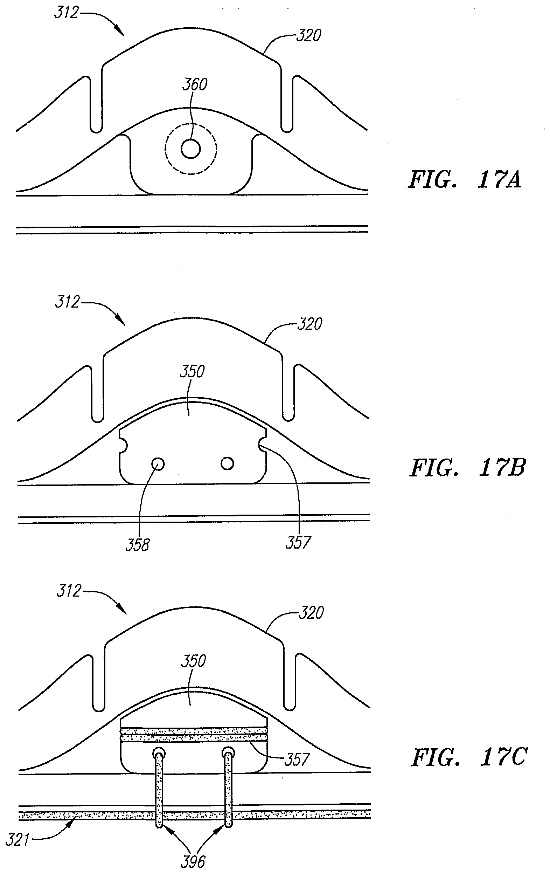

FIGS. 17A-17C are details of a sewing cuff core, showing a method for attaching the connector of FIGS. 16A-16C to the sewing cuff core.

### DETAILED DESCRIPTION OF THE PREFERRED EMBODIMENTS

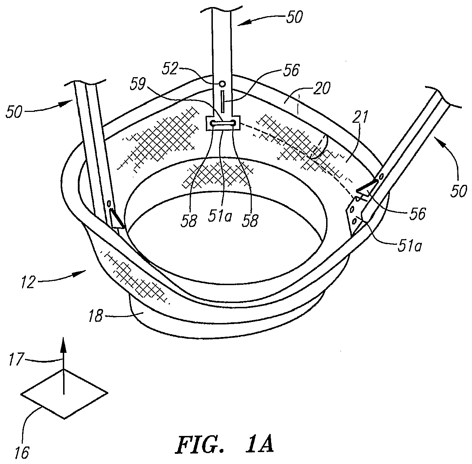

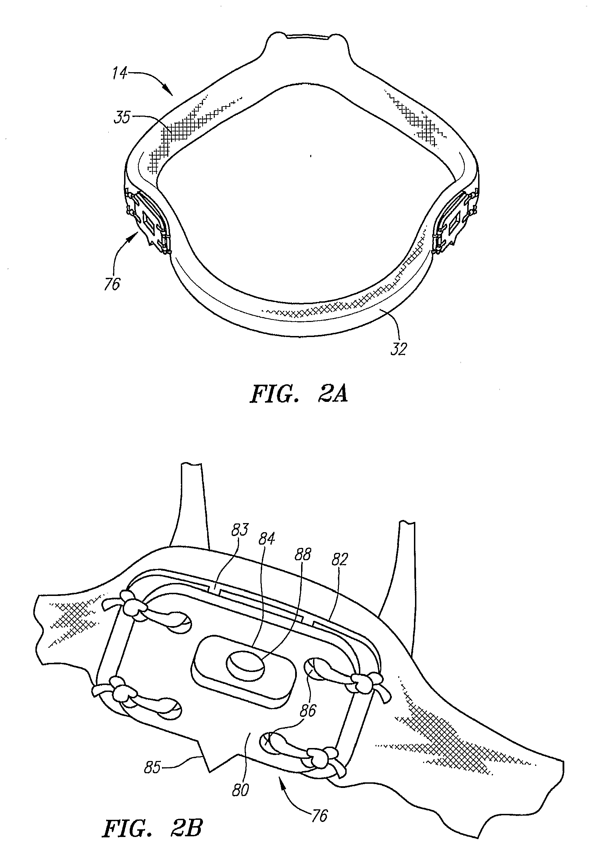

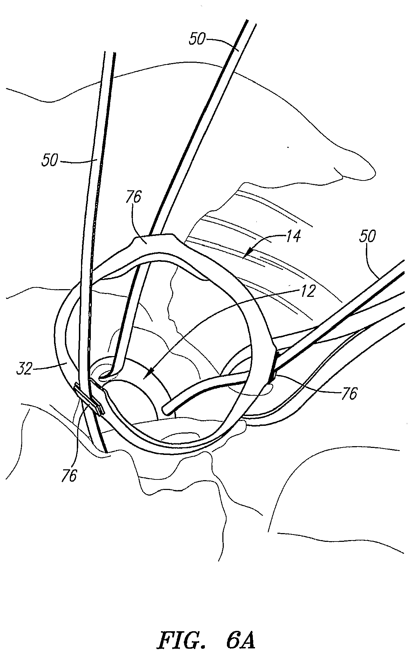

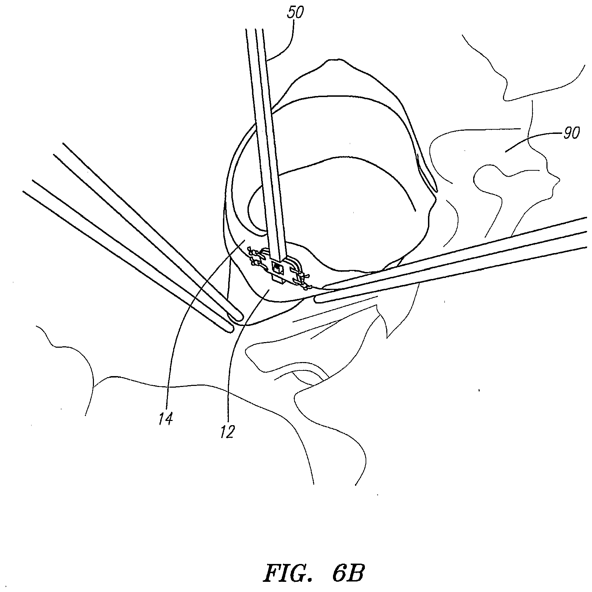

Turning to the drawings, FIGS. 1A and 2A show an embodiment of a gasket member 12 and a valve member 14, respectively, that may be combined to provide a heart valve assembly 10, e.g., as shown in FIGS. 6A and 6B.

As shown in FIG. 1A, the gasket member 12 generally includes an annular ring 18, a sewing cuff 20, and a plurality of guide rails or other elements 50 extending from the sewing cuff 20 or other portion of the gasket member 12, as described further below. Optionally, the gasket member 12 may also include a flexible skirt and/or baleen elements (not shown), e.g., surrounding a lower portion of the annular ring 18. A fabric covering 21 may be provided on one or more components of the gasket member 12, e.g., over the annular ring 18 and/or over a core of the sewing cuff 20.

In one embodiment, the annular ring 18 may have a generally circular shape generally parallel to plane 16, and/or may include an undulating shape relative to longitudinal axis 17. Alternatively, the annular ring 18 may have a multi-lobular shape about the circumference, including lobes separated by scallops or cusps (not shown). In addition or alternatively, the annular ring 18 may be expandable and/or contractible such that the diameter (or other cross-section if the annular ring 18 is noncircular may be adjusted, e.g., based upon the anatomy of the patient encountered during a procedure. In one embodiment, the annular ring 18 may be biased to expand to a predetermined diameter. Thus, the annular ring 18 may be contracted radially to a smaller diameter, e.g., to facilitate delivery into an annulus, yet may be resiliently expandable to dilate tissue surrounding the annulus and/or to facilitate securing the gasket member 12 within the annulus.

The annular ring 18 may be formed from an elastic or superelastic material, such as Nitinol, stainless steel, plastic, and the like. For example, the annular ring 18 may be cut from a flat sheet of base material having a desired thickness for the annular ring 18, e.g., between about 0.1-0.5 millimeters, for example, by laser cutting, mechanical cutting, and the like. Thus, the annular ring 18 may be initially formed as a long band of material, having a width corresponding to the desired width of the annular ring 18, e.g., between about 1.5-2.5 millimeters, and a length corresponding to a desired circumference of the annular ring 18, e.g., between about 55-90 millimeters. The band may then be wrapped around a mandrel or otherwise restrained in a generally cylindrical shape with the ends adjacent to one another, and the band may be heat treated or otherwise processed to program the generally cylindrical shape to create the annular ring 18. The generally cylindrical shape may include the ends overlapping one another, spaced apart from one another to provide an open "C" shape, or attached to one another.

With continued reference to FIG. 1A, the sewing cuff 20 may extend radially outwardly from the annular ring 18, e.g., from an upper portion of the annular ring 18, as shown. The sewing cuff 20 may include a flexible core material covered by fabric, e.g., by attaching the core material to the upper portion of the annular ring 18. Alternatively, the sewing cuff 20 may simply be a layer of fabric or other material covering at least a portion of the annular ring 18.

The material of the core and/or sewing cuff 20 may be substantially flexible, e.g., manufactured in a desired annular shape (such as those just described), yet easily deformed, e.g., deflected, stretched, and/or compressed. The core may be sufficiently flexible to be "floppy," i.e., such that the sewing cuff 20 conforms easily and/or substantially based upon the particular anatomy and/or implantation arrangements encountered during implantation. Thus, when the sewing cuff 20 is placed above or within a tissue annulus within a patient's heart, the core may conform to the surrounding anatomy and/or may deform when the valve member 14 is secured to the gasket member 12, e.g. to enhance sealing between the valve member 14 and the gasket member 12.

For example, when implanted within or above a tissue annulus, the core may lie against the surrounding tissue, thereby changing its shape from its original generally circular or multi-lobular shape, changing the shape of any undulations, and/or changing the angle of the original taper. Thus, the core may become more vertical or inward when it lies against the commissures (not shown) of the tissue annulus, and become more horizontal or outward when it lies within the sinuses above and between the commissures. When fasteners (not shown) arc driven through the sewing cuff 20, the corc may resiliently stretch or compress to distribute forces from the fasteners more evenly, which may reduce bunching of the sewing cuff 20 or other distortions that may otherwise result in leakage, as explained further below.

Exemplary materials for the core include silicone or other elastomeric materials, foam, fabric, felt, polymers, and the like. The materials may be molded or otherwise formed into the core, e.g., using molding, extrusion, cutting, or other manufacturing procedures. For example, the core may be injection molded or otherwise formed in its annular shape.

Turning to FIG. 2A, the valve member 14 generally includes an annular shaped body or frame 32, a plurality of receptacles 76, and one or more valve elements (not shown). The valve member 14 may include a fabric covering 35, similar to the gasket member 12, e.g., covering the frame 32 and/or other components of the valve member 14. Many features of the gasket member 12 and/or valve member 14 may be similar to the devices disclosed in co-pending US applications Serial Nos. 10/327,821, filed December 20, 2002, 10/765,725, filed January 26, 2004, 11/069,081, filed February 28, 2005, 11/144,254, filed June 3, 2005, 11/279,246, filed April 10, 2006, and 60/746,038, filed April 29, 2006, and 11/420,720, filed May 26, 2006. For example, the valve elements may be leaflets of biological material, e.g., bovine pericardium, such as those disclosed in co-pending application Serial No. 11/144,254.

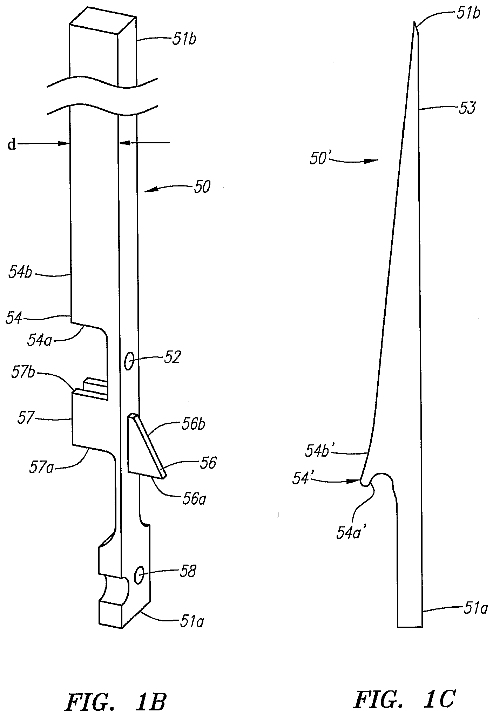

Returning to FIG. 1A, the gasket member 12 includes a plurality of elongate leaders or guide rails 50 extending from the sewing cuff 20. The guide rails 50 may be formed from elongate bands or other structures including a first end 51a attached or otherwise secured to the gasket member 12 and a second or free end 51b (not shown, see, e.g., FIG. 1C). The first ends 51a of the guide rails 50 may include apertures 58 for attaching the guide rails 50 to the gasket member 12, e.g., using one or more connectors through apertures, such as sutures (as shown in FIG. 1A), staples, clips, adhesives, and the like (not shown). For example, sutures 59 may be directed through the apertures 58 and through the fabric covering 21, e.g., to "pick up" one or more threads, thereby securing the first end 51a to the gasket member 12.

The guide rails 50 may be formed by molding, extruding, or otherwise forming elongate bands, e.g., having a rectangular cross-section. Desired features, such as those described below, may be formed into the bands, e.g., by machining, etching, cutting, drilling, or otherwise removing material from the bands. Alternatively, the desired features may be formed original in the bands, e.g., by injection molding. The guide rails 50 may be formed from materials having sufficient column strength such that the guide rails are substantially self-supporting, e.g., do not collapse under their own weight, yet are sufficiently flexible to be manipulated during use, e.g., to direct them out of the way when desired.

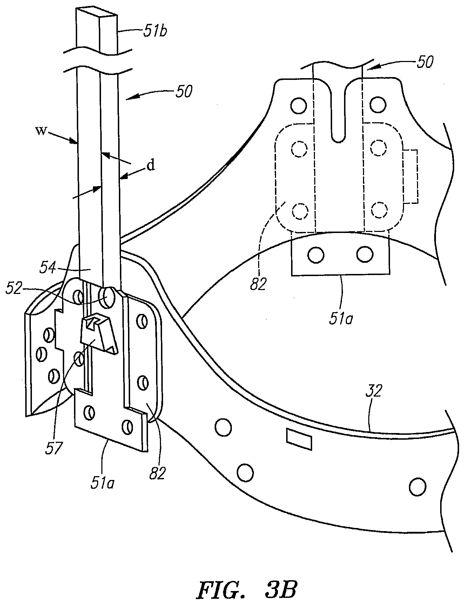

Turning to FIG. 1B, the guide rails 50 include one or more connectors 54, 56, 57 for securing the valve member 14 (not shown) to the first end 51a, and thereby to the gasket member 12. As shown, each guide rail 50 includes one or more primary locking tabs 54, 57, and one or more secondary locking tabs 56, e.g., on opposite sides of the guide rail 50. For example, the guide rail 50 includes a width "w" and a depth "d" that may be smaller than the width "w," e.g., as best seen in FIG. 3B. The locking tabs 54, 56, 57 may be integrally formed on the guide rails 50, e.g., at the time the guide rails 50 are formed, or may be separate elements (made from the same or different materials than the guide rails 50) that are bonded, fused, or otherwise attached to the guide rails 50 at predetermined locations.

As shown, a first locking tab 57 extends from an outer surface of the guide rail 50 that includes substantially blunt upper and lower edges 57a, 57b. Optionally, a second locking tab 54 may be provided above the first locking tab 57 that includes a substantially blunt lower edge 54a. The second locking tab 54, however, may have a ramped or tapered upper edge 54b, i.e., having a depth "d" that reduces or tapers towards the second end 51b of the guide rail 50. Optionally, a third locking tab 56 may be provided on an inner surface opposite the first and second locking tabs 54. As shown in FIG. 1B, the third locking tab 56 includes a blunt lower edge 56a and a tapered upper edge 56b. As explained further below, the third locking tab 56 may prevent inadvertent separation of the first and second locking tabs 54, 57 from a valve member (not shown).

In another option, the first locking tab 57 may be omitted. For example, as shown in FIG. 1C, a guide rail 50' may include a locking tab 54' on an outer surface that includes a substantially blunt lower edge 54a' and a tapered surface 54b' that extends towards the second end 51b. As shown, the locking tab 54' includes an eagle's beak or hooked end, which may enhance engagement with a valve member (not shown), as explained further below.

Optionally, the guide rails 50 may include weakened or breakaway regions to facilitate severing the guide rails 50, e.g., above the locking tabs 56, 57. For example, as best seen in FIG. 1B, each guide rail 50 may include a hole or slot 52 extending between the outer and inner surfaces above the front tab 57. Thus, with material of the guide rail 50 removed, the hole 52 may provide a weakened region that breaks preferentially when the guide rail 50 is subjected to a predetermined tensile force, as explained further below. In an exemplary embodiment, the resulting weakened region may require approximately three pounds (3 lbs.) of force to break away the lower portion of the guide rail 50 from the remaining excess portion.

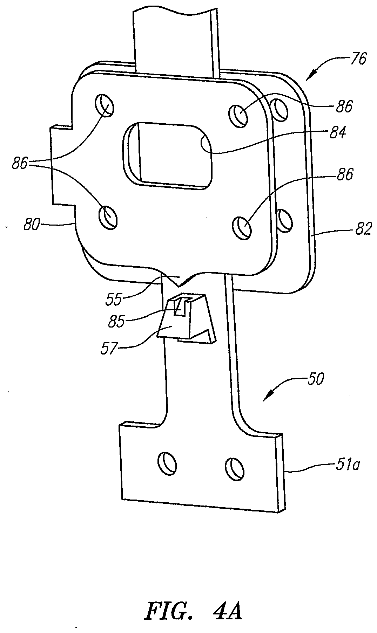

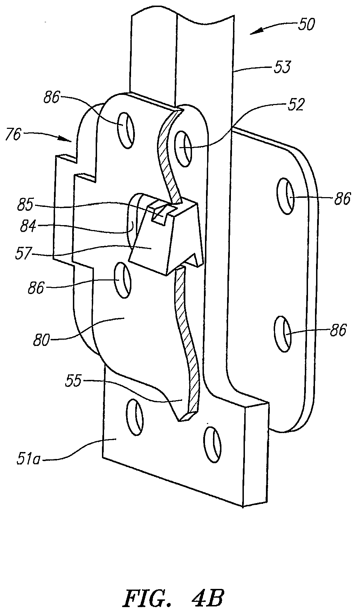

Turning to FIG. 2A, the valve member 14 may include a plurality of receptacles 76 for receiving respective guide rails 50 (not shown, see, e.g., FIGS. 4A and 4B), as described further below. As shown, a receptacle 76 may be provided at each of the commissures of the frame 32. Alternatively, the receptacles 76 may be provided at other locations around the frame 32 that correspond to locations where guide rails 50 are provided on the gasket member 12 (not shown). Although three receptacles 76 are shown, it will be appreciated that fewer or more receptacles 76 may be provided corresponding to respective guide rails 50 on the gasket member 12.

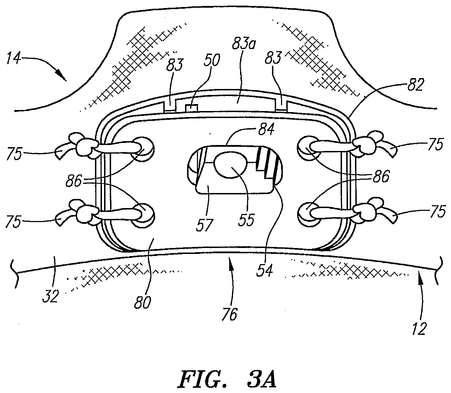

As shown in FIGS. 2B, 3A, and 3B, each receptacle 76 may be formed from front and rear locking plates 80, 82 spaced apart from one another, e.g., by one or more spacers 83 extending from one or both of the front and rear plates 80, 82. The spacer(s) 83 may also define a passage 83a between the plates 80, 82, e.g., to guide the guide rails 50 between and/or through the plates 80, 82. The front and rear plates 80, 82 may be attached to one another, e.g., using adhesives, heat bonding, cooperating detents or other connectors, and the like. Alternatively, the front and rear plates 80, 82 may be integrally formed as a single piece, e.g., by injection molding, machining, and the like.

As best seen in FIGS. 3A and 3B, the front and rear plates 80, 82 may include a plurality of holes 86 therethrough for receiving sutures 75 or other connectors therethrough, e.g., for connecting the receptacle 76 to the valve member 14. For example, sutures 75 may be directed through corresponding holes 86 in the front and rear plates 80, 82 and through the fabric of the valve member 14. The sutures 75 may then be knotted and the excess suture material cut or otherwise severed. In an alternative embodiment, the receptacles 76 may be attached directly to the frame 32, e.g., such that the receptacles 76 do not move substantially relative to the frame 32. Alternatively, other methods may be used to attach the receptacles to the valve member 14, e.g., adhesives, detents or other connectors on the frame 32, fabric, and/or receptacle 76, and the like (not shown). As shown in FIG. 3A, the holes 86 may be arranged on the front and rear plates 80, 28 such that one of the plates (e.g., the rear plate 82, as shown) is subjected to compression between opposite side edges. This may bias the front and rear plates 80, 82 slightly away from one another to open the passage 83a between the front and rear plates 80, 82, thereby facilitating directing a guide rail 50 (not shown) through the receptacle 76.

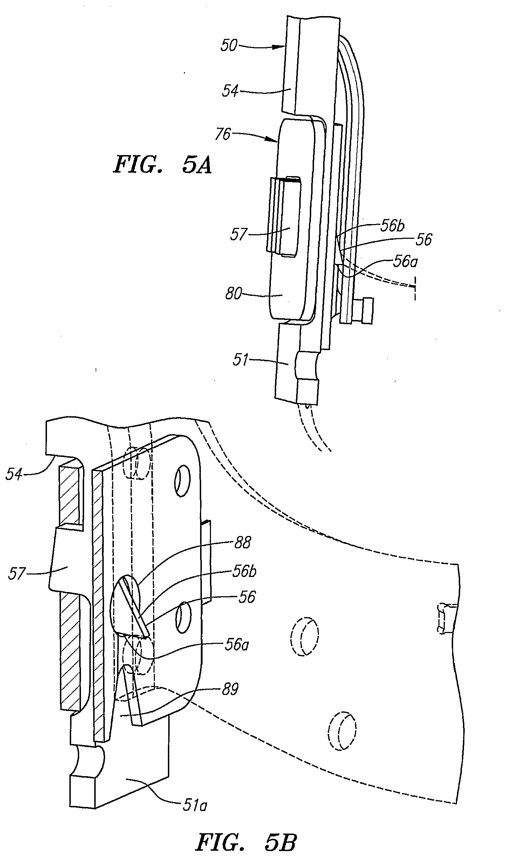

One or both of the plates 80, 82 may include one or more apertures, windows, recesses, or other pockets 84, 88 therein. As shown in FIGS. 3A, 4A, 4B, and 5A, the front plate 80 may include a front window 84 having a size and/or shape for receiving the first or front locking tab 57 therein. Similarly, as shown in FIG. 5B, the rear plate 82 may include a rear window 88 having a size and/or shape for receiving the third or rear locking tab 56 therein. Optionally, as best seen in FIG. 4A, the front plate 80 may include a tapered tooth or other element 85 extending downwardly from a lower edge of the front plate 80. In this option, the front tab 57 may include a dimple or groove 55, which may interact with the element 85, e.g., to enhance proper guiding of the front tab 57 through the receptacle 76 and into the first window 84.

Turning to FIG. 3A, a fully actuated connector is shown that is created by the interaction of a guide rail 50 and a receptacle 76. As shown, the first tab 57 is positioned within the front window 84. With additional reference to FIGS. 4A and 4B, during use, the free end 51b of the guide rail 50 is directed through the receptacle 76, i.e., from the lower end of the receptacle 76, between the plates 80, 82 and upwardly. Optionally, the free end 51b may include a narrow, tapered, and/or other shape (not shown) to facilitate directing the free end 51b through the receptacle 76.

Turning to FIG. 4A, the receptacle 76 may be directed downwardly along the guide rail 50 (e.g., when the valve member 14 is directed towards the gasket member 12, as described further below), e.g., until the ramped tooth 55 contacts the groove 85 in the front tab 57. The cooperation of the tooth 55 with the groove 85 may center the front tab 57 within the receptacle 76, thereby aligning the front tab 57 axially with the front window 84. In addition, this cooperation may lift or otherwise separate the lower edge of the front plate 80 from the rear plate 82, thereby opening the passage 83a between the plates 80, 882 to facilitate directing the front tab 57 between the plates 80, 82.

Turning to FIG. 4B, once the first tab 57 is fully aligned with and/or enters the front window 84, the plates 80, 82 are free to resiliently return back towards one another. Thus, the blunt upper edge 57b of the front tab 57 may engage the blunt upper edge 84b of the front window 84. Similarly, as shown in FIGS. 5A and 5B, the rear plate 82 may include a tab centering groove 89. As the receptacle 76 is directed downwardly over the guide rail 50, the rear tab 56 may enter the groove 89, thereby further centering or otherwise guiding the receptacle 76 along the guide rail 50. The tapered upper edge 56b of the rear tab 56 may facilitate the rear tab 56 passing under the rear plate 82 and into the receptacle 76 until the rear tab 56 enters the rear window 88. The blunt lower edge 56a of the third tab 56 also prevents the receptacle 76 from moving upwardly along the guide rail 50, e.g., to enhance securing the valve member 14 to the gasket member 12, as described further below.

Turning to FIGS. 6A and 6B, a method is shown for implanting a prosthetic heart valve assembly 10 into a biological annulus. Generally, the heart valve assembly 10 includes a gasket member 12 and a valve member 14, such as that shown in FIGS. 1A and 2A and/or as described elsewhere herein or in the applications identified elsewhere herein. The biological annulus 90 may be the site for replacing an existing natural or previously implanted heart valve, such as a tricuspid, mitral, aortic, or pulmonary valve within a patient's heart (not shown).

Before implanting the heart valve assembly of FIGS. 1A and 2A, the patient may be prepared for the procedure using known methods. For example, the patient may be placed on cardiopulmonary bypass (CPB), and the patient's heart may be exposed, e.g., by sternotomy, thoracotomy, or other open or minimally invasive procedure. An incision may be created in the blood vessel above the valve being replaced (not shown), e.g., in the aorta for an aortic valve replacement, in order to access the annulus 90. The existing natural or prosthetic heart valve and/or leaflets (also not shown) may then be removed from the annulus 90 using known methods.

A gasket member 12 and a valve member 14 may be selected based upon the anatomy encountered, e.g., having a plurality of lobes matching the lobes of the biological annulus 90 and/or having a cross-sectional dimension corresponding to the interior cross-section of the biological annulus 90. Optionally, a gasket member 12 and/or valve member 14 may be selected having a size that is larger than the biological annulus 90. For example, the gasket member 12 may have a diameter in its relaxed condition that is slightly larger than the biological annulus 90, e.g., such that the gasket member 12 may at least partially dilate the biological annulus 90 upon implantation. In addition or alternatively, the valve member 14 may have a diameter or other cross-section that is substantially larger than the biological annulus 90, e.g., for supra-annular or intra-sinus implantation, which may accommodate the larger size.

With reference to FIG. 6A, the gasket member 12 may be introduced into the patient until the annular ring 18 (not shown in FIG. 6A) is disposed within the biological annulus. The gasket member 12 may be restrained in a contracted condition by tensioning guide rails 50, e.g., with a delivery tool (not shown) and introduced into the patient's body until the annular ring 18 extends at least partially into the biological annulus 90. The gasket member 12 may then be expanded or at least partially released within the biological annulus 90, e.g., to dilate the biological annulus 90 or otherwise direct the surrounding tissue outwardly. Once stabilized, the guide rails 50 may be released entirely from the delivery tool (not shown). Optionally, a dilation tool (not shown) may be advanced into the gasket member 12 and expanded to forcibly (e.g., plastically) expand the annular ring (not shown) within the biological annulus 90. In an alternative embodiment, a tool (not shown) may be used to maintain the gasket member 14 in the contracted condition, and the gasket member 14 may be released once the annular ring 18 is positioned within the biological annulus 90, whereupon the gasket member 14 may resiliently expand, e.g., to contact and/or dilate tissue surrounding the annulus 90. Such a delivery tool may also constrain or limit movement of the guide rails 50 during delivery of the gasket member 18, e.g., to keep the guide rails 50 substantially out of the field of view.

With the gasket member 12 deployed within the biological annulus 90, the sewing cuff 20 may contact the tissue surrounding the supra-annular space above the biological annulus 90. One or more fasteners, e.g., clips or sutures (not shown), may be directed through the gasket member 12 into the tissue above and/or surrounding the biological annulus 90. Exemplary fasteners and methods for using them to secure the gasket member 12 may be found in co-pending Application Serial Nos. 10/327,821, filed 20 December 2002, 10/646,639, filed 22 August 2003, 10/681,700, filed 8 October 2003, and 11/004,445, filed December 3, 2004.

Turning to FIG. 6B, with the gasket member 12 within the biological annulus 90, the valve member 14 may then be advanced into the patient's body towards the biological annulus 90. In the embodiment shown, the valve member 14 may be advanced along the guide rails 50 toward the gasket member 12. Before advancing the valve member 14, the free ends 51b of the guide rails 50 may be directed through respective receptacles 76 of the valve member 14. Thus, before advancing the valve member 14, the guide rails 50 need to be released and removed completely from any delivery tool (not shown) if used to deliver the gasket member 12.

With the guide rails 50 received through the receptacles 76 of the valve member 14, the valve member 14 may be advanced distally over the guide rails 50 towards the gasket member 12 until the valve member 14 engages or otherwise contacts the gasket member 12. For example, the valve member 14 may be advanced until the receptacles 76 securely engage with locking tabs 54, 56, 57, e.g., until the locking tabs 57, 56 are received in respective windows 84, 88 in the receptacle 76. Consequently, as shown in FIG. 6B, the valve member 14 is securely positioned relative to gasket member 12, with the locking tabs 54, 56, 57 preventing the valve member 14 from being moved away from the gasket member 12.

The excess portions of the guide rails 50 above the receptacles 76 may then be removed. For example, the free ends 51b of the guide rails 50 may be pulled with sufficient tensile force to break the weakened regions on the guide rails 50. Alternatively, the guide rails 50 may simply be cut or otherwise severed above the valve member 14.

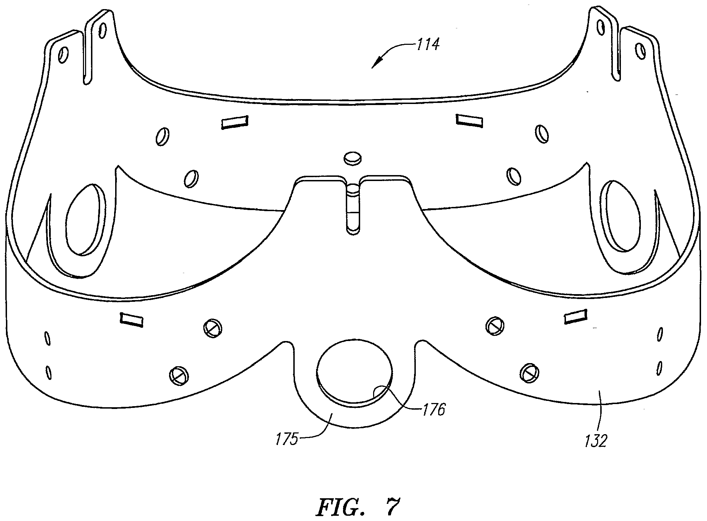

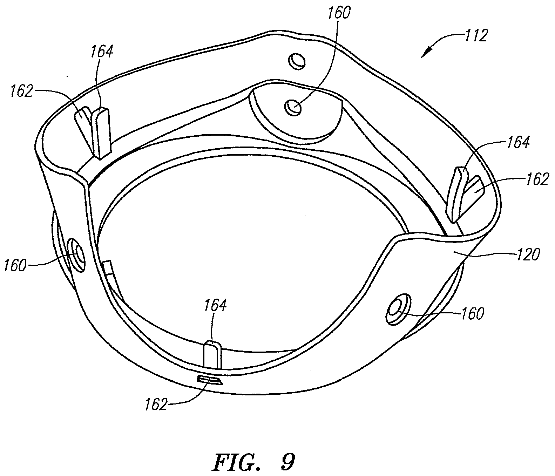

Turning to FIGS. 7-12, an alternative embodiment of a heart valve assembly 100 is shown that includes a gasket member 112 (only a portion of which is shown in FIGS. 9, 11, and 12) and a valve member 114 (the leaflets and fabric being omitted for clarity). Generally, as described above, the gasket member 112 includes a sewing cuff extending radially from an annular ring, and a fabric covering (not shown for clarity). An exemplary core 120 for the sewing cuff is shown in FIGS. 9, 11, and 12. Also similar to previous embodiments, the valve member 114 includes a frame 132, one or more valve elements, and a fabric covering (not shown).

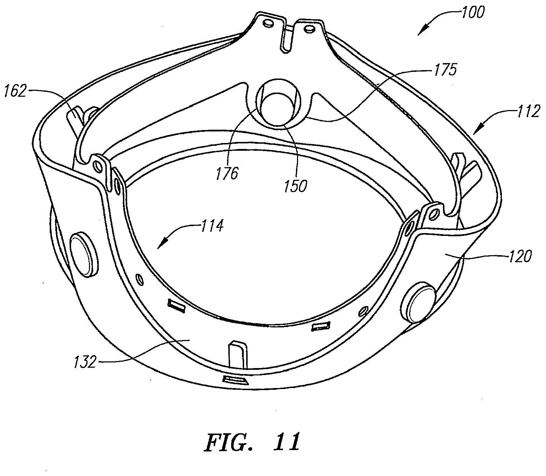

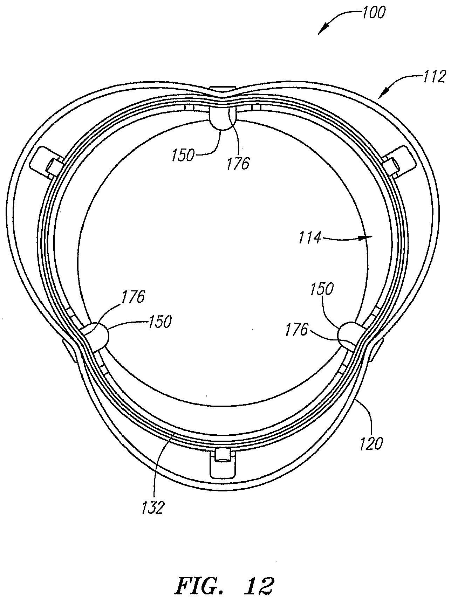

Turning to FIG. 9, the flexible corc 120 for the sewing cuff of the gasket member 112 may be formed from silicone or other resilient, flexible material, as described elsewhere herein and in the applications identified elsewhere herein. The flexible core 120 may include a plurality of connector holes 160 therein for receiving respective connectors, such as studs (not shown, see, e.g., FIGS. 11 and 12). Optionally, the flexible core 120 may include a plurality of closable windows 162. For example, the flexible core 120 may be cut to create the windows 162 including tabs 164 therein. Each tab 164 may include one end fixed to the flexible core 120 and a free end selectively receivable in the respective window 162. In one embodiment, the tabs 164 may have their shape set, e.g., such that the tabs 164 extend away from the respective windows 162. Yet, when the tabs 164 are directed into the respective windows 162, there may be sufficient interference to maintain the tabs 164 in the windows 162. Thus, during use, the tabs 164 may be pressed into the windows 162 to close the windows 162 or may be directed out of the windows 162 to provide openings for receiving sutures, staples, clips, or other connectors (not shown) through the windows 162.

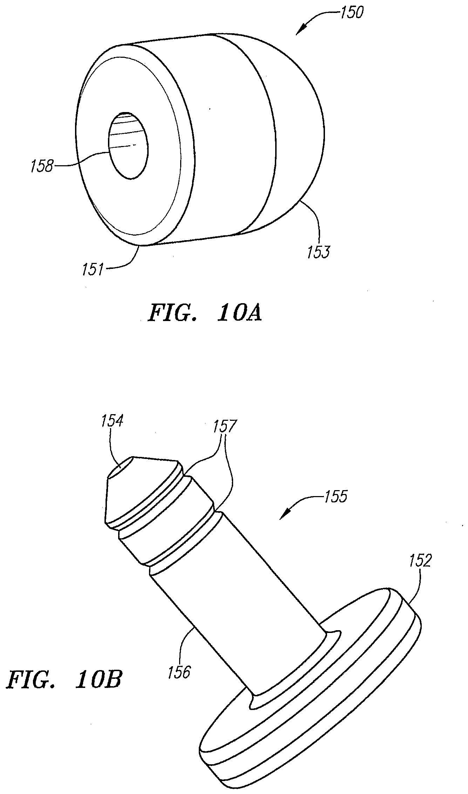

Turning to FIGS. 10A and 10B, a connector is shown that includes a cap 150 and a base 155, respectively. As shown in FIG. 10A, the cap 150 includes a flat first end 151 having a pocket 158 therein and a rounded second end 153. As shown in FIG. 10B, the base 155 includes a shaft 156 extending from an enlarged head 152 and terminating in a tip 154. The shaft 156 has a size and/or shape for being received in the pocket 158 in the cap 150, e.g., to provide an interference fit therebetween. Optionally, the shaft 156 may include one or more annular grooves 157 and/or the cap 150 may include one or more annular ridges, tabs, or other detents (not shown) within the pocket 158.

Turning to FIGS. 9 and 11, to attach the connector to the flexible core 120, the shaft 156 of the base 155 is inserted through the connector holes 160 from the outside of the flexible core 120 until the tip 154 is exposed within the interior of the flexible core 120. The cap 150 may then be directed over the tip 154 such that the tip 154 is received in the pocket 158. The cap 150 may be secured to the shaft 156 by an interference fit, by the grooves 157 engaging corresponding ridges or tabs (not shown), and/or using adhesives, fusing, or other methods.

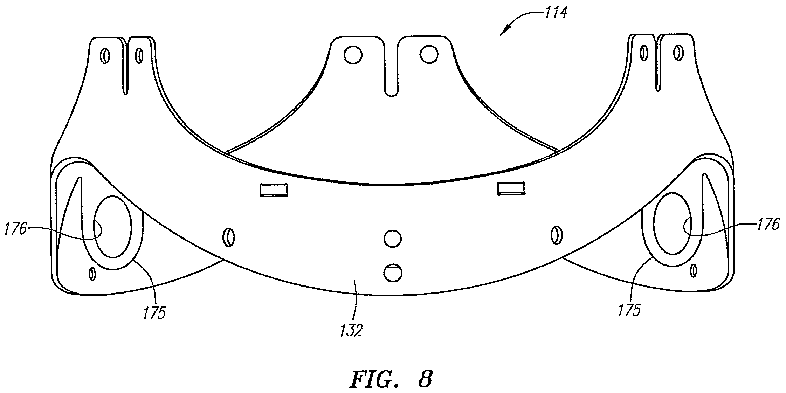

Turning to FIGS. 7 and 8, the valve member 114 includes a plurality of receptacles, e.g., locking windows 176, extending from the frame 132. For example, the frame 132 may include tabs or other extensions 175 formed from the frame material that extend downwardly from the frame 132. The tabs 175 include holes or openings 176 therethrough, e.g., cut or otherwise created through the tabs 175. The openings 176 may be sized and/or shaped to receive the connectors 150, 155 therethrough, as described further below. The locations of the tabs 175 around the circumference of the frame 132 may correspond to the locations of the connectors 150 on the flexible core 120, e.g., at the commissures.

Implantation of the heart valve assembly 100 may proceed as described elsewhere herein. For example, a gasket member (not shown) including the flexible core 120 of FIGS. 9, 11, and 12 may be directed into a biological annulus and secured therein. For example, one or more clips, sutures, or other connectors (not shown) may be directed through the windows 162 in the flexible core 120 and into surrounding tissue. A valve member including the frame 132 may be directed into the biological annulus towards the gasket member until tabs 175 are disposed above the connectors 150. For example, guide rails (not shown) may be provided on the gasket member (e.g., similar to the guide rails 50 without the locking tabs 54, 56, 57), and the valve member may be advanced down the guide rails. Alternatively, the valve member may simply be carried by a valve holder and directed into the annulus towards the gasket member.

As the valve member is directed further towards the gasket member, the tabs 175 may contact the connectors 150, causing the connectors 150 to deform or deflect until the connectors 150 enter the windows 176. Once the connectors 150 enter the windows 176, the connectors 150 may resiliently return outwardly, thereby creating an interference fit between the connectors 150 and the tabs 175. The connectors 150 may be formed from resiliently deflectable material, such as plastic. The rounded end 153 of the cap may facilitate directing the connectors 150 out of the way until the connectors 150 are freely exposed within the windows 176.

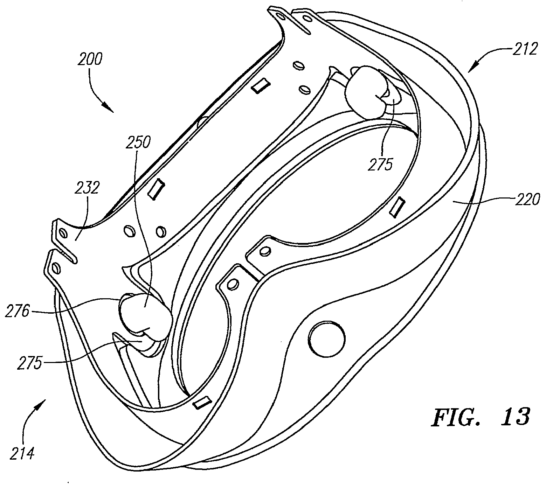

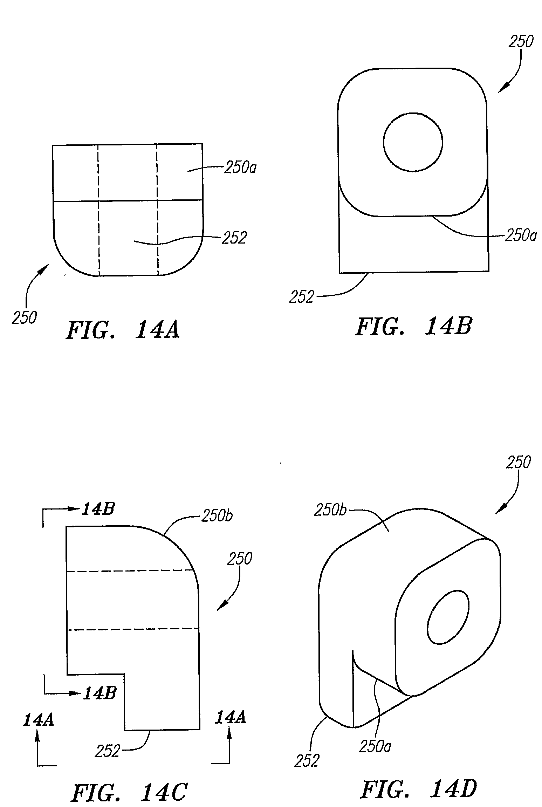

Turning to FIG. 13, an alternative example for understanding the invention of a heart valve assembly 200 is shown that includes a gasket member 212 (with the annular ring and fabric covering omitted for clarity) and valve member 214 (with the leaflets and fabric covering also omitted for clarity), similar to those described above. The valve member 214 includes a frame 232, which may include receptacles, e.g., tabs 275 with openings or windows 276, similar to the embodiment shown in FIGS. 7-12. Unlike the previous embodiments, the gasket member 212 includes connectors, such as studs 250 shown in FIGS. 14A-14D, which may include rounded upper surfaces 250b and hooked lower surfaces 250a, e.g., to enhance engagement with the windows 276 in the tabs 275.

Turning to FIGS. 14A-14D, the connector or stud 250 may include a hooked or "eagle's beak" shape, e.g., including a blunt lower surface 250a and a curved or rounded upper surface 250b. As shown, the lower surface 250a includes a hook element 252 extending therefrom. The connector 250 may be connected to a flexible core or other portion of the gasket member 212, similar to the embodiments described elsewhere herein. The connector 250 may be formed from a deformable material, e.g., an elastomer or other plastic, which allows the connector to deform downwardly and/or inwardly, yet resiliently return outwardly to its original shape.

During use, the gasket member 212 may be secured within a biological annulus, and the valve member 214 may be directed towards the gasket member 212 until the tabs 275 contact the connectors 250. The rounded upper surfaces of the connectors 250 allow the tabs 275 to be directed downwardly, deforming or bending the connectors 250, until the connectors 250 are aligned with the windows 276, whereupon the connectors 250 may resiliently return outwardly to engage the connectors 250 within the windows 276. The blunt lower surfaces 250a may contact the lower edges of the windows 276, preventing subsequent removal of the connectors 250. The hooked element 252 may enhance engagement, e.g., preventing the connectors 250 from being removed from the windows 275 even if the connectors 250 subsequently move and/or are deformed, and consequently, preventing the valve member 214 from being separated from the gasket member 212.

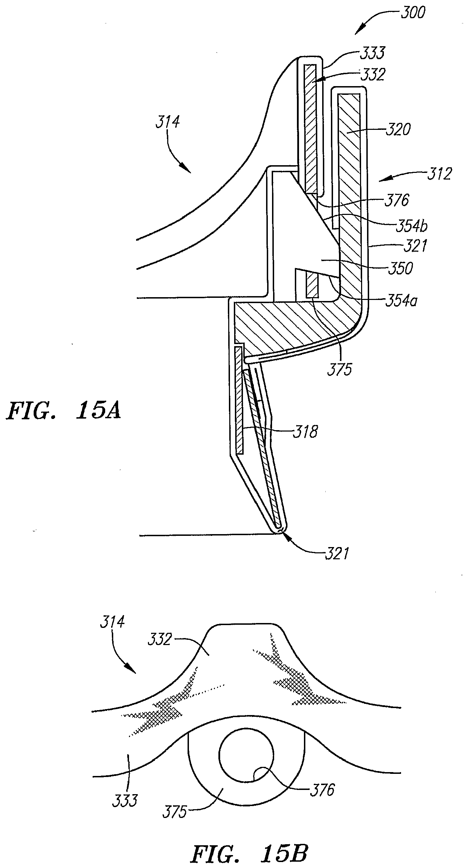

Turning to FIG. 15A, a cross-sectional view of yet another alternative example for understanding the invention of a heart valve assembly 300 is shown that generally includes a gasket member 312 and valve member 314, similar to the previous embodiments. For example, the gasket member 312 may include an annular ring 318, a sewing cuff 320, and a fabric covering 321, and the valve member 314 may include a frame 332, a fabric covering 333, and one or more valve elements (not shown). The valve member 314 may also include one or more connectors, e.g., tabs 375 with windows 376 extending from the frame 332, similar to previous embodiments. FIG. 15B is a detail of the valve member 314 including the fabric covering over the frame 332, but with the tab 375 and window 376 exposed.

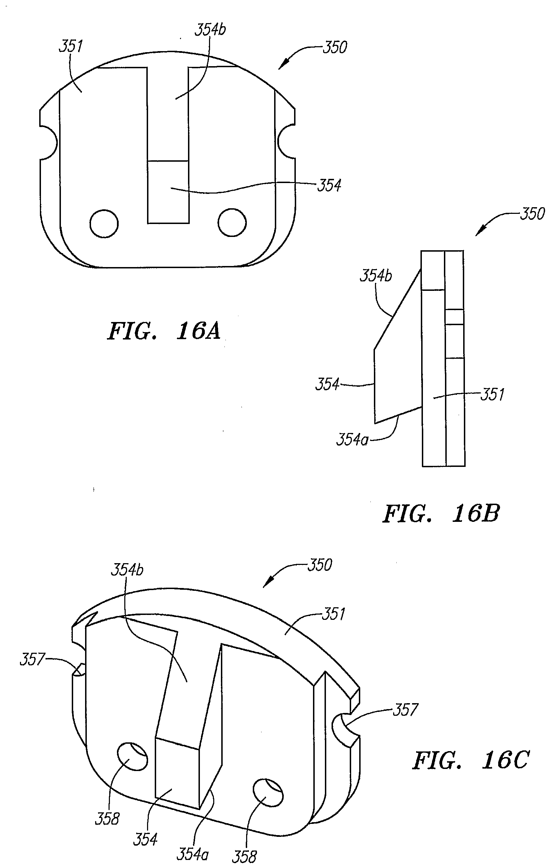

Returning to FIG. 15A, the gasket member 312 includes a plurality of buckles 350 (one shown) that may interlock with a respective tab 375 and window 376 to secure the valve member 314 relative to the gasket member 312. Turning to FIGS. 16A-16C, the buckle 350 may include a planar portion 351 and a tab 354 extending outwardly from the planar portion 351. Optionally, the buckle 350 may include other features, such as one or more grooves 357 and/or apertures 358, e.g., for receiving sutures (not shown). As shown, the tab 354 includes a substantially blunt lower surface 354a and a ramped or tapered upper surface 354b.

As shown in F1GS.17A-17C, the buckle 350 may be attached to the gasket member 312, e.g., to a core 320 of the sewing cuff, e.g., using sutures 396. As shown, the buckle 350 may be disposed adjacent an interior surface of the gasket member 312 with the tab 354 oriented outwardly, e.g., as shown in FIG. 15A. Sutures 396 may be directed through the apertures 357, 358 and through the sewing cuff 320 and/or fabric covering 321 of the gasket member 312, thereby securing the buckle 350 with the tab 354 abutting the sewing cuff 320.

The heart valve assembly 300 may be implanted within a biological annulus similar to the other embodiments described herein. However, when the tabs 375 on the frame 332 contact the respective tabs 354 of the buckle 350, the ramped upper surfaces 354b of the tabs 354 may direct or deflect the buckles 350 inwardly, allowing the tabs 375 to pass between the buckle 350 and the sewing cuff 320. Once the tabs 375 pass below the tabs 354, the tabs 354 may enter the respective windows 376, and the buckle 350 may then resiliently move outwardly, thereby capturing the tabs 375 between the buckle 350 and the sewing cuff 320. The blunt lower edges 354a of the tabs 354 may prevent the tabs 375 from moving upwardly, thereby securing the valve member 314 to the gasket member 312. Optionally, the tabs 354 may include a hook or eagle's beak shape (not shown) to enhance engagement, similar to the embodiments described above.

## Claims

1. A prosthesis (12) for receiving a prosthetic valve (14, 114, 214) to replace a preexisting natural or prosthetic heart valve within a biological annulus (90), comprising:
an annular member (18) implantable within the biological annulus (90);
a sewing cuff (20) extending outwardly from the annular member;
a plurality of guide rails (50) comprising first ends (51a) coupled to one of the annular member and the sewing cuff for guiding a prosthetic valve (14, 114, 214) towards at least one of the sewing cuff and the annular member; **characterised by** one or more locking tabs (54, 56, 57) positioned on the guide rails (50) for securing the prosthetic valve (14, 114, 214) relative to the prosthesis (12).

2. A heart valve assembly implantable within a biological annulus, comprising the prosthesis (12) according to claim 1, further comprising:
a second prosthesis (14, 114, 214) comprising an annular frame (32, 132), at least one valve element and receptacles (76) for receiving respective guide rails (50) such that the second prosthesis may be directed along the guide rails towards the first prosthesis (12); wherein the
locking tabs (54, 56, 57) on the guide rails (50) are for engaging respective windows (84, 88) in the receptacles (76) for securing the second prosthesis (14, 114, 214) relative to the first prosthesis (12).

3. The heart valve assembly of claim 2, wherein the locking tabs (54, 56, 57) comprise first and second locking tabs extending from opposite sides of the guide rails (50).

4. The heart valve assembly of claim 3, wherein the receptacles (76) comprise first and second pockets (84, 88) for receiving the first and second locking tabs, respectively, therein.

5. The heart valve assembly of claim 4, wherein the receptacles (54, 56, 57) comprise first and second plates (80, 82) spaced apart from one another, the first and second plates comprising the first and second pockets (84, 88), respectively, therein.

6. The heart valve assembly of claim 8, wherein the locking tabs (54, 56, 57) further comprise a third tab spaced apart above the first tab.

7. The heart valve assembly of claim 6, wherein the first tab (57) comprises substantially blunt upper and lower surfaces, and wherein the third tab (56) comprises a substantially blunt lower surface (56a) and a tapered upper surface (56b).

8. The heart valve assembly of claim 2, wherein each of the guide rails includes a weakened region above the one or more locking tabs (54, 56, 57) for detaching an upper portion of the guide rail (50) from a lower portion.

9. The heart valve assembly of claim 2, wherein the first ends (51a) of the guide rails (50) are attached to the sewing cuff (20) and are spaced apart from one another about a periphery of the sewing cuff.

10. The heart valve assembly of claim 2, wherein the guide rails (50) are sufficiently rigid to be self-supporting.

11. The heart valve assembly of claim 10, wherein the guide rails (50) are sufficiently flexible to be manipulated by a user to direct second free ends (51b) of the guide rails toward or away from one another.

12. The heart valve assembly of claim 2, wherein the guide rails (50) have a rectangular cross-section wherein optionally the cross-section of the guide rails (50) define a width that extends along a periphery of the sewing cuff (20) and a depth that extends transversely relative to the periphery, the depth being smaller than the width.

13. The heart valve assembly of claim 2, wherein the receptacle (76) comprisesmeans for engaging with locking tabs (54, 56, 57).

14. A heart valve assembly implantable within a biological annulus, comprising the prosthesis (12) of claim 1, further comprising:
a second prosthesis (14, 114, 214) comprising an annular frame (32, 132), at least one valve element
and receptacles (76) for receiving respective guide rails (50) such that the second prosthesis may be directed along the guide rails (50) towards the first prosthesis (12); and
means for securing the second prosthesis (14, 114, 214) relative to the first prosthesis (12) once the second prosthesis is directed along the guide rails (50) towards the first prosthesis (12).

## Patentansprüche

1. Prothese (12) zum Aufnehmen einer prothetischen Klappe (14, 114, 214) zum Ersetzen einer bereits vorhandenen natürlichen oder prothetischen Herzklappe in einem biologischen Annulus (90), wobei die Prothese Folgendes umfasst:
ein ringförmiges Element (18), das in den biologischen Annulus (90) implantiert werden kann;
einen Nähbund (20), der sich von dem ringförmigen Element aus nach außen erstreckt;
eine Vielzahl von Führungsschienen (50), die erste Enden (51a) umfassen, die mit einem des ringförmigen Elements und des Nähbunds verbunden sind, um eine prothetische Klappe (14, 114, 214) in Richtung mindestens eines des Nähbunds und des ringförmigen Elements zu führen; **gekennzeichnet durch**
eine oder mehrere Verschlusslaschen (54, 56, 57), die an den Führungsschienen (50) positioniert sind, um die prothetische Klappe (14, 114, 214) relativ zur Prothese (12) zu sichern.

2. Herzklappenanordnung, die in einen biologischen Annulus implantiert werden kann und die Prothese (12) nach Anspruch 1 umfasst und ferner Folgendes umfasst:
eine zweite Prothese (14, 114, 214), die einen ringförmigen Rahmen (32, 132), mindestens ein Klappenelement und Aufnahmeelemente (76) zum Aufnehmen jeweiliger Führungsschienen (50) umfasst, sodass die zweite Prothese entlang der Führungsschienen in Richtung der ersten Prothese (12) geführt werden kann; wobei die
Verschlusslaschen (54, 56, 57) an den Führungsschienen (50) zum Aufnehmen jeweiliger Fenster (84, 88) in den Aufnahmeelementen (76) zum Sichern der zweiten Prothese (14, 114, 214) relativ zur ersten Prothese (12) dienen.

3. Herzklappenanordnung nach Anspruch 2, wobei die Verschlusslaschen (54, 56, 57) eine erste und eine zweite Verschlusslasche umfassen, die sich von gegenüberliegenden Seiten der Führungsschienen (50) aus erstrecken.

4. Herzklappenanordnung nach Anspruch 3, wobei die Aufnahmeelemente (76) eine erste und eine zweite Aussparung (84, 88) jeweils zum Aufnehmen der ersten bzw. der zweiten Verschlusslasche darin umfassen.

5. Herzklappenanordnung nach Anspruch 4, wobei die Aufnahmeelemente (54, 56, 57) voneinander beabstandete erste und zweite Platten (80, 82) umfassen, wobei die erste und die zweite Platte jeweils die erste bzw. die zweite Aussparung (84, 88) darin umfassen.

6. Herzklappenanordnung nach Anspruch 8, wobei die Verschlusslaschen (54, 56, 57) ferner eine dritte Lasche umfassen, die beabstandet über der ersten Lasche angeordnet ist.

7. Herzklappenanordnung nach Anspruch 6, wobei die erste Lasche (57) eine im Wesentlichen glatte Ober- und Unterseite umfasst und wobei die dritte Lasche (56) eine im Wesentlichen glatte Unterseite (56a) und eine sich verjüngende Oberseite (56b) umfasst.

8. Herzklappenanordnung nach Anspruch 2, wobei jede der Führungsschienen einen abgeschwächten Bereich über der einen oder den mehreren Verschlusslaschen (54, 56, 57) zum Lösen eines oberen Abschnitts der Führungsschiene (50) von einem unteren Abschnitt beinhaltet.

9. Herzklappenanordnung nach Anspruch 2, wobei die ersten Enden (51a) der Führungsschienen (50) am Nähbund (20) befestigt sind und beabstandet zueinander um einen Umfang des Nähbunds herum angeordnet sind.

10. Herzklappenanordnung nach Anspruch 2, wobei die Führungsschienen (50) steif genug sind, um sich selbst zu tragen.

11. Herzklappenanordnung nach Anspruch 10, wobei die Führungsschienen (50) flexibel genug sind, dass sie von einem Benutzer bewegt werden können, um zweite freie Enden (51b) der Führungsschienen zueinander hin oder voneinander weg zu führen.

12. Herzklappenanordnung nach Anspruch 2, wobei die Führungsschienen (50) einen rechteckigen Querschnitt aufweisen, wobei optional der Querschnitt der Führungsschienen (50) eine Breite definiert, die sich entlang eines Umfangs des Nähbunds (20) erstreckt, und eine Tiefe, die sich quer zum Umfang erstreckt, wobei die Tiefe geringer ist als die Breite.

13. Herzklappenanordnung nach Anspruch 2, wobei die Aufnahmeelemente (76) Mittel zum Eingreifen mit den Verschlusslaschen (54, 56, 57) umfassen.

14. Herzklappenanordnung, die in einen biologischen Annulus implantiert werden kann und die Prothese (12) nach Anspruch 1 umfasst und ferner Folgendes umfasst:
eine zweite Prothese (14, 114, 214), die einen ringförmigen Rahmen (32, 132), mindestens ein Klappenelement und Aufnahmeelemente (76) zum Aufnehmen jeweiliger Führungsschienen (50) umfasst, sodass die zweite Prothese entlang der Führungsschienen (50) in Richtung der ersten Prothese (12) geführt werden kann; und
Mittel zum Sichern der zweiten Prothese (14, 114, 214) relativ zur ersten Prothese (12), sobald die zweite Prothese entlang der Führungsschienen (50) in Richtung der ersten Prothese (12) geführt wurde.

## Revendications

1. Prothèse (12) pour recevoir une valve prothétique (14, 114, 214) pour remplacer une valve cardiaque préexistante naturelle ou prothétique dans un anneau biologique (90), comprenant :
un élément annulaire (18) implantable dans l'anneau biologique (90) ;
un manchon de suture (20) s'étendant vers l'extérieur à partir de l'élément annulaire ;
une pluralité de rails de guidage (50) comprenant des premières extrémités (51a) couplées à l'un de l'élément annulaire et au manchon de suture pour guider une valve prothétique (14, 114, 214) vers au moins l'un des manchons de suture et de l'élément annulaire ; **caractérisée par** une ou plusieurs languettes de verrouillage (54, 56, 57) positionnées sur les rails de guidage (50) pour fixer la valve prothétique (14, 114, 214) par rapport à la prothèse (12) .

2. Ensemble de valve cardiaque implantable dans un anneau biologique, comprenant la prothèse (12) selon la revendication 1, comprenant en outre :
une seconde prothèse (14, 114, 214) comprenant un cadre annulaire (32, 132), au moins un élément de valve et des réceptacles (76) pour recevoir des rails de guidage (50) respectifs de sorte que la seconde prothèse puisse être dirigée le long des rails de guidage vers la première prothèse (12) ; dans lequel les
languettes de verrouillage (54, 56, 57) sur les rails de guidage (50) sont destinées à engager des fenêtres (84, 88) respectives dans les réceptacles (76) pour fixer la seconde prothèse (14, 114, 214) par rapport à la première prothèse (12) .

3. Ensemble de valve cardiaque selon la revendication 2, dans lequel les languettes de verrouillage (54, 56, 57) comprennent des première et deuxième languettes de verrouillage s'étendant à partir des côtés opposés des rails de guidage (50).

4. Ensemble de valve cardiaque selon la revendication 3, dans lequel les réceptacles (76) comprennent des première et seconde poches (84, 88) pour recevoir les première et deuxième languettes de verrouillage, respectivement, dans ceux-ci.

5. Ensemble de valve cardiaque selon la revendication 4, dans lequel les réceptacles (54, 56, 57) comprennent des première et seconde plaques (80, 82) espacées les unes des autres, les première et seconde plaques comprenant les première et seconde poches (84, 88) respectivement, dans ceux-ci.

6. Ensemble de valve cardiaque selon la revendication 8, dans lequel les languettes de verrouillage (54, 56, 57) comprennent en outre une troisième languette espacée au-dessus de la première languette.

7. Ensemble de valve cardiaque selon la revendication 6, dans lequel la première languette (57) comprend des surfaces supérieure et inférieure sensiblement émoussées, et dans lequel la troisième languette (56) comprend une surface inférieure sensiblement émoussée (56a) et une surface supérieure conique (56b).

8. Ensemble de valve cardiaque selon la revendication 2, dans lequel chacun des rails de guidage comprend une région affaiblie au-dessus des une ou plusieurs languettes de verrouillage (54, 56, 57) pour détacher une partie supérieure du rail de guidage (50) d'une partie inférieure.

9. Ensemble de valve cardiaque selon la revendication 2, dans lequel les premières extrémités (51a) des rails de guidage (50) sont fixées au manchon de suture (20) et sont espacées l'une de l'autre autour d'une périphérie du manchon de suture.

10. Ensemble de valve cardiaque selon la revendication 2, dans lequel les rails de guidage (50) sont suffisamment rigides pour être autoporteurs.

11. Ensemble de valve cardiaque selon la revendication 10, dans lequel les rails de guidage (50) sont suffisamment flexibles pour être manipulés par un utilisateur afin de diriger les secondes extrémités libres (51b) des rails de guidage vers ou s'éloignant l'une de l'autre.

12. Ensemble de valve cardiaque selon la revendication 2, dans lequel les rails de guidage (50) possèdent une section transversale rectangulaire dans laquelle, facultativement, la section transversale des rails de guidage (50) définit une largeur qui s'étend le long d'une périphérie du manchon de suture (20) et une profondeur qui s'étend transversalement par rapport à la périphérie, la profondeur étant inférieure à la largeur.

13. Ensemble de valve cardiaque selon la revendication 2, dans lequel le réceptacle (76) comprend des moyens pour s'engager avec des languettes de verrouillage (54, 56, 57).

14. Ensemble de valve cardiaque implantable dans un anneau biologique, comprenant la prothèse (12) selon la revendication 1, comprenant en outre : une seconde prothèse (14, 114, 214) comprenant un cadre annulaire (32, 132), au moins un élément de valve et des réceptacles (76) pour recevoir des rails de guidage (50) respectifs de telle sorte que la seconde prothèse puisse être dirigée le long des rails de guidage (50) vers la première prothèse (12) ; et des moyens pour fixer la seconde prothèse (14, 114, 214) par rapport à la première prothèse (12) une fois que la seconde prothèse est dirigée le long des rails de guidage (50) vers la première prothèse (12).
